# EUROPÄISCHE PATENTSCHRIFT

(11) **EP 2 887 907 B1**
(45) Veröffentlichungstag und Bekanntmachung des Hinweises auf die Patenterteilung: **31.07.2019**
(21) Anmeldenummer: 13782937.0
(22) Anmeldetag: 21.08.2013
(51) Int. Cl.: A61F 2/16

(54) **INTRAOKULARLINSE, INSBESONDERE ZILIARINTRAOKULARLINSE**
INTRAOCULAR LENS, IN PARTICULAR CILIARY INTRAOCULAR LENS
LENTILLE INTRAOCULAIRE, NOTAMMENT LENTILLE INTRAOCULAIRE CILIAIRE

(30) Priorität: 24.08.2012 DE 102012016892
(43) Veröffentlichungstag der Anmeldung: 01.07.2015
(73) Patentinhaber: Biolnic UG, 50937 Köln (DE)
(72) Erfinder: JANSEN, Josef, D-51429 Bergisch-Gladbach (DE)
(74) Vertreter: Patentanwälte Vomberg & Schart
(86) Internationale Anmeldenummer: PCT/DE2013/000471
(87) Internationale Veröffentlichungsnummer: WO 2014/029382

(56) Entgegenhaltungen:
- US-A1- 2003 149 480
- US-A1- 2004 082 993
- US-A1- 2008 161 913
- US-B1- 6 443 985

## Beschreibung

Die vorliegende Erfindung betrifft eine Ziliarintraokularlinse mit mindestens einer Optik und einer Haptik. Ferner betrifft die Beschreibung ein Verfahren zur Implantation einer Intraokularlinse mit mindestens einer Optik, einer Haptik sowie einer Füllung. Schließlich betrifft die Beschreibung eine Intraokularlinse mit mindestens einer Optik, einer Haptik sowie einer zumindest im optischen Bereich befindlichen Füllung.

Die sogenannte Akkommodation ist die dynamische Anpassung der Brechkraft des Auges. Bei der Nahsicht befindet sich die natürliche Linse in ihrem kugeligen, unverformten und unbelasteten Zustand, in der keine Kräfte auf sie wirken. Der Ziliarmuskel ist dabei angespannt und konzentrisch verengt und die Zonulafasern sind schlaff. Entspannt sich der Ziliar (Fernakkommodation), straffen sich die Zonula und spannen den Kapselsack über seinen Äquator in radialer Richtung. Der Kapselsack übt dadurch eine axiale Druckkraft auf die Linse aus, wodurch diese sich in eine weniger gekrümmte Ellipse verformt, womit die Fernsicht ermöglicht wird. Bei Kontraktion des Ziliar (Nahakkommodation) geht die Linse aufgrund ihrer Eigenelastizität in ihre kugeligere Eigenform zurück, was wieder mit einer Zunahme der Brechkraft einhergeht. Der Durchmesser von Ziliar und Linse sowie die Dicke der Linse ändern sich zwischen Nah- und Fernsichtjeweils um etwa 0,5 mm. Die Elastizität der Linse lässt mit zunehmendem Alter nach und führt schließlich zur Presbyopie (Alterssichtigkeit). Die Korrektur erfolgt in der Regel mit einer Brille.

Eine besonders gravierende und langsam fortschreitende Alterserscheinung ist ferner die Katarakt, bei der es zur Trübung der Augenlinse kommt. Die Folgen einer solchen Katarakt sind erhöhte Blendempfindlichkeit des Auges und die verblasste Wahrnehmung von Farben. Die fortgeschrittene Katarakt kann nur operativ behandelt werden. Hierzu wird zunächst die Linse mittels Ultraschall zertrümmert, abgesaugt und anschließend eine künstliche, meistens aufgerollte, Intraokularlinse über einen kleinen Schnitt durch die Hornhaut in den geöffneten Kapselsack injiziert. Die nach dem Stand der Technik bekannten Intraokularlinsen sind monofokal, womit sie nur einen Brennpunkt besitzen. Mittels sogenannter Haptiken werden die Optiken der Intraokularlinsen üblicherweise im Kapselsack zentriert. Asphärische Optiken verbessern durch die Vermeidung von Streulicht das Kontrast- und Nachtsehen, während Intraokularlinsen mit besonderen (UV-)Filtern die Netzhaut schützen. Materialien mit einem hohen Brechungsindex sind ferner von Vorteil, um bei gleicher Dioptrienstärke dünnere Optiken bzw. Intraokularlinsen für kleinere Inzisionen fertigen zu können. Kleinere Schnitte müssen nicht mehr mit einer Naht verschlossen werden. Zudem wird die Wahrscheinlichkeit einer postoperativen Hornhautverkrümmung (Astigmatismus) erheblich gesenkt.

Daneben sind nach dem Stand der Technik Bifokal- bzw. Multifokallinsen bekannt. Der Nachteil der Multifokallinsen liegt in dem deutlich schlechteren Kontrastsehvermögen und erhöhter Blendempfindlichkeit.

Die häufigste Komplikation bei der Verwendung von Intraokularlinsen ist der postoperative Nachstar. Dieser entsteht zum größten Teil durch Proliferation restlicher oder regenerierter Linsenepithelzellen, die nach extrakapsulärer Kataraktextraktion im Kapselsack verblieben sind. Der Nachstar erfordert eine Laserbehandlung, die mit möglichen Komplikationen verbunden sein kann. Eine faltenfreie Ausspannung der hinteren Kapsel gilt als eine mögliche wirksame Nachstarprophylaxe.

Trotz zahlreicher Versuche ist es bisher nicht gelungen, die Akkommodation des Auges mit ausreichender Brechkraftänderung über einen längeren Zeitraum wiederherzustellen.

Die meisten Konzepte zur Wiederherstellung der Akkommodation sehen die Implantation einer Intraokularlinse in den Kapselsack ("Kapselintraokularlinsen") vor.

Dabei können zwei grundsätzliche Konzepte unterschieden werden, nämlich Intraokularlinsen nach dem sogenannten "Optic-Shift-Prinzip" und die Linsenkapselwiederauffüllung ("lens refilling") mit flüssigen bzw. viskosen Materialien. Die Linsenkapselwiederauffüllung hat sich aufgrund verschiedener Probleme jedoch nicht durchsetzen können.

Bei dem Optic-Shift-Prinzip werden ein oder zwei Optiken längs der optischen Achse der Intraokularlinsen verschoben. Eine alleinige Verschiebung der Optik auf der optischen Achse erreicht jedoch keine zufrieden stellende Akkommodationsleistung, da der Verschiebeweg begrenzt ist.

Daneben gehören auch Intraokularlinsen zum Stand der Technik, die nicht in den Kapselsack implantiert werden und deren Haptik unmittelbar in Kontakt zum Ziliar steht (sog. Ziliarintraokularlinsen). Zur Implantation wird der Kapselsack zuvor entfernt oder liegt zumindest teilweise nach der Implantation posterior der Intraokularlinse. Solche Intraokularlinsen können im Sulcus der hinteren Augenkammer eingesetzt oder am Ziliarmuskel oder auch der Sklera befestigt werden.

Der wesentliche Vorteil einer Ziliarintraokularlinse gegenüber einer Kapselintraokularlinse liegt in einem deutlich höheren Kraftübertragungspotential, hervorgerufen durch die direkte Anbindung an den Ziliar, die zu einer deutlich höheren Akkommodationsleistung der Intraokularlinse führen kann.

Beispielsweise wird in der US 4892543 eine solche Ziliarintraokularlinse offenbart, deren 2 U-förmigen Haptikarme nur zu einem sehr geringen Umfangsbereich in Verbindung mit dem Ziliar stehen und daher nur eine begrenzte radiale Kraftübertragung ermöglichen.

Ein weiteres Beispiel ist in DE 10346024A1 beschrieben, wo ein Distanzring die Intraokularlinse hält und zentriert, der in den Sulcus bzw. um den Ziliarmuskel geformt ist. Diese Konstruktion ermöglicht nur eine Verschiebung und keine Formänderung der Optiken.

In der US 2009/0012609A1 wird eine Ausführungsform einer Ziliarintraokularlinse beschrieben, bei der sich sowohl die Dicke als auch die Krümmung der Optiken ändem sollen. Eine ausreichende radiale Kraftübertragung ist hier allerdings aus den gleichen bereits beschriebenen Gründen nicht möglich.

Weitere Intraokularlinsen werden in US 2003/149480 A1, US 2008/161913 A1 und US 2004/082993 A1 offenbart:, die ebenfalls jeweils eine Optik und eine die Optik umgreifende Haptik aufweisen.

Die Aufgabe der vorliegenden Erfindung ist es, eine Intraokularlinse zu schaffen die eine symmetrische Verformung einer oder mehrerer Optiken der Intraokularlinse sowie eine relative Verschiebung dieser Optiken auf ihrer optischen Achse zueinander zu ermöglichen, so dass eine ausreichende Brechkraftänderung erzielt wird.

Diese Aufgabe wird durch die Ziliarintraokularlinse nach Anspruch 1 gelöst, wonach erfindungsgemäß vorgesehen ist, dass die Haptik aus mehreren Haptikelementen besteht, die vorzugsweise äquiangular mit der Optik verbunden sind, wobei
a) die Haptikelemente einen in einer Draufsicht im Wesentlichen trapezförmigen Teilbereich besitzen und die Basen zweier benachbarter Haptikelemente am Übergang zu der Optik miteinander verbunden sind und
b) die Haptikelemente an der der Optik abgewandten Seite der trapezförmigen Teilbereiche je ein teilringförmiges Haptikringsegment besitzen, die eine zylinderförmige Außenkontur bilden und im Querschnitt V-förmig ausgestaltet sind, so dass sich die Intraokularlinse der Kontur des Ziliar formschlüssig anpasst, um ein Verrutschen der Optik oder der Intraokularlinse innerhalb des Ziliar zu vermeiden, wobei die Haptikringsegmente zweier benachbarter Haptikelemente im unbelasteten Zustand geringfügig voneinander beabstandet sind,
so dass zwischen zwei benachbarten Haptikelementen eine im Wesentlichen tortenstück- oder strichförmige Ausnehmung ausgebildet ist, wobei im unbelasteten Zustand die kumulierte Breite der Ausnehmungen am Außendurchmesser des Haptikrings weniger als 25 % des Umfangs beträgt.

Durch die erfindungsgemäße Ausgestaltung wird erreicht, dass sowohl der Ziliar im belasteten und im unbelasteten Zustand eine größtmögliche Anlagefläche zu den Haptikringsegmenten aufweist, als auch dass die Haptik eine größtmögliche Anlagefläche zu der Optik besitzt, womit sowohl über den Umfang der Haptikringsegmente als auch über den Umfang der Optik eine gleichmäßige Kraftverteilung bei der Akkommodation geschaffen wird. Hierdurch wird die Optik gleichmäßig verformt und/oder gleichmäßig verschoben, womit Abbildungsfehler vermieden werden.

Vorteilhafte Ausgestaltungen der vorliegenden Erfindung werden im Folgenden sowie in den Unteransprüchen beschrieben.

Nach einer ersten bevorzugten Ausführungsform der Erfindung ist vorgesehen, dass die Intraokularlinse mindestens eine anteriore und eine posteriore Optik aufweist, die über die Haptik miteinander verbunden sind, wobei durch die anteriore und posteriore Optik sowie die Haptik ein Hohlraum gebildet wird und zumindest der optische Bereich des Hohlraums eine Füllung aufweist. Alternativ ist auch vorgesehen, dass der Bereich des Hohlraums im Bereich der Haptik und/oder der Ausnehmungen teilweise oder vollständig mit der Füllung gefüllt ist. Bei der Akkommodation ändert zumindest eine der Optiken ihre Form. Die Eigenform oder der Fertigungszustand ist vorzugsweise eine flache Form für die Fernsicht.

Die Füllung ist nach einer bevorzugten Ausführungsform der Erfindung flüssig, gelförmig oder gasförmig und weist nach einer besonders bevorzugten Ausführungsform Nanopartikel auf. Die Füllung dient dabei der Erhöhung der Akkommodationsleistung, wozu die Füllung der Intraokularlinse vorzugsweise einen höheren Brechungsindex als das Kammerwasser besitzt. Zudem ist das Medium oder Material der Füllung weicher bzw. elastischer als die Schale. Die Füllung liegt vorzugsweise vollflächig an den beiden äußeren Optiken an. Die Innenflächen der Optiken können hierbei beliebig geformt sein, insbesondere wenn Optiken und Füllung und ggf. Säckchen einen gleichen Brechungsindex haben. Die Füllung kann den Innenraum der Intraokularlinse vollständig ausfüllen oder auf den Bereich der Optiken beschränkt sein. Erstreckt sich die Füllung über den optischen Bereich in die Haptik und ist diese nicht flüssig oder gasförmig, ist die Füllung vorzugsweise im Bereich der Haptik wie die Haptik selbst mit Durchbrechungen versehen, insbesondere, wenn die Füllung mit der Haptik verbunden ist. Die Gestaltung aus gelartiger weicher Füllung und härterer Schale ermöglicht bei entsprechender Dickenverteilung der Schale und geeigneten Elastizitätsmodulen die kontrollierte Formänderung der Intraokularlinsen, um eine adäquate optische Abbildung in Nah- und Fernzustand und bis in den Randbereich der Optiken zu erzielen. Es lassen sich so auch bei weit geöffneter Iris Streulichtprobleme vermeiden bzw. deutlich reduzieren. Eine solche Formänderung der Intraokularlinse lässt sich nicht erzielen, wenn die Füllung nur von einer sehr dünnen oder biegeschlaffen Membran oder Hülle umschlossen wäre.

Die Füllung kann innerhalb der Intraokularlinse isoliert vorliegen. Wenn das Medium der Füllung flüssig ist, bietet es sich an, die Füllung innerhalb eines Säckchens bestehend aus einer sehr dünnen Membran zu integrieren. Alternativ kann die Füllung nach außen durch eine Membran vom Rest der Intraokularlinse bzw. des Kammerwassers getrennt sein. Die Membran spannt sich hierbei vorzugsweise zwischen den jeweiligen Basen der anterioren zur posterioren Haptik, also zwischen den Randbereichen der Optiken. Die Membran kann sich dabei radial nach außen ausdehnen, um so auch eine Volumenänderung zwischen Fern- und Nahsicht auszugleichen. Eine andere Möglichkeit der Abdichtung des Hohlraums besteht darin, die Durchbrechungen der Haptiken mit einer dünnen Membran zu verschließen. Die Membran oder das Säckchen, die den Innenraum radial nach außen abschließt, ist im Vergleich zur Schale einer Intraokularlinse wesentlich dünner und sollte in der Größenordnung von etwa einem Zehntel der Dicke der Schale liegen. Die Dicke einer solchen Membran liegt vorzugsweise zwischen 5 µm und 50 µm.

Die Füllung oder das mit der Füllung gefüllte Säckchen ist vorzugsweise vollflächig oder auch nur teilweise mit einer oder gleichzeitig mit beiden Optikflächen verbunden oder kann auch nur lose im Innern der Intraokularlinse liegen. Damit können zwischen den äußeren Optiken und der Füllung auch Zwischenräume entstehen, die sich ggf. mit Kammerwasser füllen. Zudem könnte die Füllung auch geteilt sein, so dass mittig ein Spalt bzw. Zwischenraum entsteht und jeweils die geteilten Füllungen mit den äußeren Optiken verbunden sind. Das Säckchen ist über die Durchbrechungen in das Innere der Intraokularlinse einführbar.

Nach einer alternativen Ausgestaltung besteht die Füllung aus einem hydrophilen Material (Hydrogel), so dass die Intraokularlinse im trockenen Zustand und mit einem kleineren Volumen leichter implantiert werden kann. Nach der Implantation in das Auge nimmt die Füllung Wasser aus dem Kammerwasser auf und nimmt die für die optische Funktion der Intraokularlinse angedachte Größe und Form an. Für diese konstruktive Auslegung sollte die ggf. vorhandene zuvor beschriebene abtrennende Membran oder auch die Intraokularlinse selbst wasserdurchlässig sein. Hierzu könnte die Membran beispielsweise perforiert sein. Alternativ oder additiv hierzu könnte nur die Haptik perforiert oder mittels Diffusion wasserdurchlässig sein, so dass das Kammerwasser in den Hohlraum eindringen kann.

Mit einer flüssigen oder gelartigen Füllung kann die Intraokularlinse ebenfalls in einem kleineren Zustand implantiert werden, wenn der Hohlraum erst nach der Implantation gefüllt wird. Es ist daher nach einer bevorzugten Ausführungsform der Erfindung vorgesehen, dass ein oder mehrere mit der Füllung gefüllte Reservoire über einen Schlauch vorzugsweise lösbar mit dem Hohlraum verbunden sind, so dass bei einer Volumenänderung des Hohlraums während der Akkommodation oder bei dem Zusammenfalten der Intraokularlinse für die Implantation die Füllung in das Reservoir überführbar ist und umgekehrt.

Nach einer bevorzugten Ausführungsform ist das Reservoir schlauchförmig ausgebildet und vorzugsweise über ein Mikroventil oder über eine Kanüle bzw. Hohlnadel mit dem Hohlraum verbunden. Hierbei könnte das schlauchförmige Reservoir über das Ende des Ventils wie ein Wasserschlauch über ein Absperrventil gestülpt sein. Alternativ kann das Reservoir auch über einen Schlauch mit dem Hohlraum verbunden sein, der schmelzend abtrennbar ist. Oder das schlauchförmige Reservoir könnte mittels einer Kanüle, die das offene Ende des Reservoirs bildet, mit dem Hohlraum verbunden sein. Nach Entfernen der Kanüle verschließt sich die Punktionsstelle wieder. Zudem könnte die Punktionsstelle in Form eines Gummipfropfs oder Nippels ("zu durchstechender Gummiverschluss") verstärkt sein. Vorteilhaft für ein dichtes Verschließen der Punktion ist, dass die für eine solche Intraokularlinse benötigten sehr weichen Polymere zumeist sehr klebrig sind. Ferner könnte optional das Ventil zwischen Kanüle und Reservoir geschaltet sein. Die Verbindungs- bzw. Schnittstelle zwischen dem Hohlraum und dem Reservoir ist vorzugsweise an der Membran, der Haptik oder außerhalb des optischen Bereichs der Optik angeordnet. Ggf. ist der Hohlraum zusätzlich mit einem Entlüftungsschlauch verbunden, um eine etwaige Blasenbildung wirksam zu vermeiden. Der Entlüftungsschlauch kann analog zu dem Reservoir mit dem Hohlraum verbunden sein. Die Vorteile des lösbaren Reservoirs zum Füllen des Hohlraumes sind, dass dem Ophthalmologen ein mit definierter Füllmenge vorgefülltes und vormontiertes Intraokularlinsensystem geliefert werden kann. Insgesamt werden so fehlerbehaftete manuelle Vorgänge, wie z.B. nicht korrekte Füllmengen und/oder Kontaminationen reduziert.

Anders ausgedrückt kann die Befüllung des Hohlraums mit der Füllung mittels eines oder ggf. mehrerer (Mikro-) Zuführschläuche und eines ggf. notwendigen Entlüftungsschlauches erfolgen, die mit dem Hohlraum verbunden sind und nach Füllung des Hohlraums mit der Flüssigkeit durch Mikroventile oder Klemmen oder Schmiernippel verschlossen oder beispielsweise mittels Verschweißen abgetrennt werden.

Die Füllung des Hohlraumes könnte auch über eine Mikrodosierpumpe erfolgen. Diese Vorgänge können außerhalb des Auges erfolgen. Die nach Verschließen des Schlauches vorhandenen Schlauchreste, die mit dem Hohlraum der Intraokularlinse verbunden sind, können ins Augeninnere und ggf. in den verbliebenen Hohlraum zwischen Haptik und Membran gezogen werden. Die Füllung könnte zudem auch über eine Spritze injiziert werden. Der Füllvorgang könnte auch zum Anpassen der Brechung, also der Dioptrien-Zahl genutzt werden.

Eine andere Möglichkeit, die Intraokularlinse mit möglichst kleinem Volumen in das Auge zu implantieren, besteht darin, das Säckchen bzw. die Füllung erst nach der Implantation über die Haptiköffnungen in das Innere der Intraokularlinse einzuführen.

In einer weiteren Ausgestaltung der Erfindung ist ein schlauchförmiges Gebilde an die Intraokularlinse angekoppelt, welches insbesondere für den Implantationsvorgang als Reservoir für die Füllung der Intraokularlinse genutzt werden kann. Hierzu ist ein speziell geformter Schlauch mit dem einen Ende mit dem Hohlraum bzw. der Füllung verbunden, am anderen Ende verschlossen. Hiermit ist die Flüssigkeit des Intraokularlinseninneren hydraulisch (fluidisch) mit dem Reservoir verbunden. Vorzugsweise ist das Reservoir entlang des Haptikringes anterior oder besonders bevorzugt posterior geführt. Zur Implantation wird die Intraokularlinse beispielsweise zunächst zusammengedrückt, so dass das Medium aus dem Inneren der Intraokularlinse in den Schlauch gepumpt und dabei der Schlauch aufgebläht wird, und anschließend zusammengerollt. Nach der Injektion und der Entfaltung der Intraokularlinse fließt das Medium zurück in den Hohlraum der Intraokularlinse. Ein Ventil in der Schlauchzuführung oder ein sonstiges Verschließen des Schlauches könnte dabei ein ungewolltes und/oder zu starkes Zurückfließen aus dem Innern in den Schlauch verhindern. Auch kann der Schlauch in seiner Funktion als Reservoir im "normalen Betrieb" genutzt werden, indem es Volumenänderungen der Füllung der Intraokularlinse während der Akkommodation ausgleicht. Das geschlossene Ende des Schlauches kann auch eine Art Blasenform annehmen.

In einer alternativen Ausführung ist das Reservoir mit der abtrennenden Membran oder dem Säckchen im Innern verbunden. Der Schlauch kann hierzu insbesondere über eine der Haptiköffnungen nach außen geführt sein, so dass dieser sich außerhalb der Intraokularlinse während der Implantation aufblähen kann. Nach Implantation kann der Schlauch in der Augenkammer verbleiben oder er kann, insbesondere im Falle der Verwendung als Kapselintraokularlinse ins Innere der Intraokularlinse gezogen und in Umfangsrichtung um die Füllung gelegt werden. Der Schlauch bzw. das Reservoir liegt dann zwischen der Haptik und der die Füllung abschließenden Membran oder Säckchens.

Die beschriebenen Ausführungsformen sind auch bei Intraokularlinsen vorgesehen, die mehrere Reservoire besitzen, die ggf. auch unterschiedlich voneinander ausgestaltet sein können.

Die Schläuche haben vorzugsweise einen Durchmesser zwischen 0,1 mm und 1 mm, besonders bevorzugt zwischen 0,3 mm und 0,8 mm, und eine Länge bis etwa 35 mm. Der Bereich des Schlauches, der ggf. als Reservoir ausgebildet sein soll, kann auch größere Durchmesser z. B. in einer Blasenform annehmen und dünnwandiger als der Rest Schlauches ausgestaltet sein. Wie bereits ausgeführt wurde, können auch mehrere Schläuche verteilt über die Intraokularlinse mit dem Inneren verbunden sein.

Die Intraokularlinse besteht vorzugsweise aus zwei im wesentlichen (von anterior nach posterior) konvex-konkav bzw. konkav-konvex geformten Halbschalen mit an den Polen integrierten Optiken, die an ihrem Äquator bzw. ihrer Haptik miteinander verbunden sind. Zweifach gekrümmte Halbschalen lassen sich nur mit relativ hohen Kräften in weniger gekrümmte oder ebene Halbschalen verformen. Daher weist der haptische Bereich der Intraokularlinse vom Äquator radial bis zur Optik ausgerichtete Ausnehmungen auf, wodurch die für eine Brechkraftänderung benötigten Verformungskräfte der Schalen deutlich reduziert werden. Die Ausnehmungen öffnen nach einer bevorzugten Ausführungsform den Innen- bzw. Hohlraum der Intraokularlinse, so dass Durchbrechungen gebildet werden, die den Hohlraum radial nach außen öffnen. Ohne an Kontraktionsvermögen einzubüßen, ist nach einer bevorzugten Ausführungsform vorgesehen, dass die Durchbrechungen durch eine Membran oder eine Haut verschlossen sind.

Vorzugsweise sind die Ausnehmungen innerhalb der Haptik vom Äquator ausgehend radial geführt und
a) enden innerhalb der Haptik,
b) führen bis zum Äquator der Optiken oder
c) über den Äquator der Optiken hinaus.

Bei den Varianten a) und b) kann der Hohlraum je nach Ausgestaltung der Haptik offen oder geschlossen ausgebildet sein. Bei der Variante c) wird der Hohlraum geöffnet, wobei die Ausnehmungen auch bis auf eine dünne Membran eingebracht werden können, womit der Hohlraum ebenfalls geschlossen bleibt.

Um eine möglichst gleichmäßige Kraftverteilung entlang des Umfangs der Optiken zu ermöglichen, liegt der Ziliar im belasteten Zustand vollständig an den Haptikringsegmenten an. Im Unbelasteten Zustand entsteht daher zwischen den Haptikringsegmenten ein minimaler Spalt bzw. eine minimale Ausnehmung, so dass der Ziliar im unbelasteten Zustand nicht vollständig an den Haptikringsegmenten anliegt. Im Falle von strichförmigen Ausnehmungen bilden sich trapezförmige oder strichförmige Haptikelemente. Nach der beanspruchten Ausführungsform beträgt die kumulierte Breite der Ausnehmungen am Außendurchmesser des Haptikrings im unbelasteten Zustand weniger als 25% und besonders bevorzugt weniger als 2-15% des Umfangs. Um ein Verrutschen der Optik bzw. der Intraokularlinse innerhalb des Ziliar zu vermeiden, bilden die Haptikringsegmente eine zylinderförmige Außenkontur und sind im Querschnitt V-förmig ausgestaltet, so dass sich dieser der Kontur des Ziliar möglichst formschlüssig anpasst und/oder sich zudem möglichst weit in den Sulcus einführen lässt. Die Haptik kann sich ferner auch nur am Ziliar abstützen, wobei ggf. zusätzliche Ärmchen oder Häkchen in den Sulcus führen und/oder die Haptik am Ziliar befestigt wird. Um eine noch effizientere Kraftübertragung zu ermöglichen, ist vorgesehen, dass der mit dem Ziliar in Kontakt stehende Bereich mit einer dünnen und weichen feinfibrillären oder porösen Struktur bedeckt ist, so dass umliegende Zellen dort einwachsen können und zu einem festen Verbund zwischen Ziliar und Haptik führen. Alternativ hierzu ist vorgesehen, dass der mit dem Ziliar in Kontakt stehende Bereich der Haptik selbst mikroporös ausgeführt ist. Zudem ist vorgesehen, dass über diese feinfibrilläre oder poröse Struktur die Intraokularlinse mit dem umliegenden Gewebe mittels Fäden oder selbstschließenden Clips vernäht oder verbunden ist. Eine solche kraftschlüssige Verbindung ist allerdings auch ohne die feinfibrilläre oder poröse Struktur auf der Haptik vorgeschlagen.

Analog zu der Ausgestaltung der Haptikringsegmente gehen die Basen der Haptikelemente optimalerweise am Umfang der Optiken ineinander über. Allerdings sind auch geringfügige Abstände zwischen den Basen möglich. Vorzugsweise betragen die kumulierten Abstände der Basen der Haptikelemente weniger als 25%, vorzugsweise weniger als 15%, des Umfangs des Außendurchmessers des Haptikrings im unbelasteten Zustand. Nach einer besonders bevorzugten Ausführungsform der Erfindung sind die Basen der Haptikelemente bündig mit der Optik verbunden, bei einer Intraokularlinse mit zwei Optiken ist bevorzugt zumindest eine entsprechend ausgestaltet.

Als alternative Formgestaltungen der Haptikelemente sind neben der Strichform auch T-förmige Haptikelemente möglich, die gewährleisten, dass sowohl am Äquator der Intraokularlinse als auch am Optikrand die Haptiken möglichst vollflächig mit dem jeweiligen Umfang des Ziliar bzw. der Optik ineinander übergehen, zugleich aber eine höhere Flexibilität als die Strichform aufweisen. Darüber hinaus kann sich zudem die Wandstärke der Haptiken von der Basis der Haptik aus verjüngen, um die Flexibilität weiter zu erhöhen. Außerdem entstehen durch die T-förmigen Haptikelemente größere Öffnungen (tortenstückförmige Ausnehmungen) in der Haptik, durch die die schlauchförmigen Reservoire nach außen gezogen werden können.

Um aber eine Durchmesseränderung des Ziliar möglichst vollständig in eine Durchmesseränderung der Intraokularlinse, hierbei insbesondere der optischen Bereiche übertragen zu können und um damit eine möglichst hohe Brechkraftänderung zu erzielen, ist es in einer weiteren Ausgestaltung vorteilhaft die Haptik steifer auszubilden. Hierzu können die Haptik oder Teile der Haptik aus einem Material mit einem höheren Elastizitätsmodul als die Optiken geformt sein. Alternativ hierzu kann die Haptik vom Äquator zur Basis der Haptikringsegmente im Querschnitt an Dicke zunehmen, so dass dieser steifer wird und sich in radialer Richtung weniger verformen kann.

Es wurde bereits beschrieben, dass die Haptik vorzugsweise bündig mit den Optiken verbunden ist, so dass die Haptik ohne Dickensprünge in den optischen Bereich übergeht. Allerdings können auch geringe Dickenunterschiede ausgebildet sein, sofern die erforderliche Verformbarkeit der Optiken hierdurch nicht über Gebühr beeinträchtigt wird. Es hat sich gezeigt, dass bei Dickenunterschieden von 80% bis 90% die Funktionsweise noch weitgehend erhalten ist.

Bevorzugt sind die Optiken der Intraokularlinse von anterior nach posterior konvex-konkav bzw. konkav-konvex und als Sammellinsen geformt, d.h. die Optiken sind im Randbereich dünner als auf der zentralen optischen Achse. Jedoch kann auch eine der beiden Optiken, bevorzugt die posteriore, auch zum Ausgleich von patientenindividuellen Sehfehlern herangezogen werden und beispielsweise eine bi-konvexe Form annehmen. In diesem Fall sind auch größere Dickenunterschiede tolerabel. Nach einer besonders bevorzugten Ausführungsform der vorliegenden Erfindung besitzt die Intraokularlinse mindestens vier, vorzugsweise mindestens sechs, aber besonders bevorzugt zwölf Haptikelemente.

Die kumulierte Breite der Einschnürungen, also der schmalsten Bereiche der T-förmigen Haptikelemente, die sich beim Übergang der Haptik in den Haptikring ausbilden, ist abhängig vom Elastizitätsmodul des verwendeten Materials und beträgt mindestens 10%, vorzugsweise mehr als 25% des Umfangs auf Höhe der Haptikspitzen.

Der Außendurchmesser der Intraokularlinse insbesondere als Ziliarintraokularlinse beträgt im Fertigungs- bzw. Fernzustand 9 mm bis 13,5 mm. Der Durchmesser der Optik der Intraokularlinse beträgt dem gegenüber 3,5 mm bis 9,5 mm, wobei die Dicke an den Polen 2,5 mm bis 6 mm beträgt. Die Wandstärken der Optiken betragen abhängig von ihrem Elastizitätsmodul vorzugsweise zwischen 0,2 mm und 1,5 mm, wiederum bevorzugt zwischen 0,5 mm und 1,2 mm. Wenn eine der beiden Optiken eine konvex-konvexe Form annimmt, kann die Optik im zentralen optischen Bereich deutlich dicker werden und mehr als 2 mm Wandstärke annehmen. Die Höhe des zylinderförmigen Haptikringes, also die Höhe des Äquators, wo beide Intraokularlinsenhalbschalen übereinander liegen, beträgt zwischen 0,3 mm und 2,5 mm, vorzugsweise zwischen 0,5 und 1,5 mm. Die Breite bzw. Dicke des Haptikrings liegt zwischen 0,5 mm und 2,5 mm.

Im Falle der Verwendung der Intraokularlinse als Kapselintraokularlinse können die zuvor aufgezeigten Abmessungen auch kleiner ausfallen.

Der Durchmesser der Ziliarintraokularlinse soll bevorzugt etwas größer sein als der Durchmesser des Ziliar. Die Ziliarintraokularlinse wird so minimal vorgespannt. Dadurch kann eine maximal mögliche Kontraktion und damit maximal mögliche Durchmesseränderung für die Akkommodationsleistung genutzt werden. Die Ziliarintraokularlinse sollte daher bevorzugt unter dieser Vorspannung für den Fernzustand ausgelegt sein, d.h. dass die Intraokularlinse erst nach der durch die Vorspannung hervorgerufenen Verformung den Fernpunkt erreicht. Dieser optische Auslegungspunkt könnte auch über eine sogenannte "Negative Akkommodation" beschrieben werden, dabei wird die Intraokularlinse in einem Fernakkommodationszustand ausgelegt, der über den Fernpunkt hinausgeht. Dieser Fernpunkt liegt im Bereich von 5 m bis 200, vorzugsweise 50 m bis 150 m. Diese Auslegung der Intraokularlinse unter einer Vorspannung wird insbesondere bei Patienten bedeutsam, deren Ziliar nur eine sehr geringe Durchmesseränderung (z.B. 0,2mm) bzw. Kontraktion aufweist und bei denen dennoch bei möglichst geringer Durchmesseränderung eine möglichst hohe Brechkraftänderung erzielt werden soll.

Die Ziliarintraokularlinse wird in einem flachen Zustand für die Fernsicht so ausgelegt, dass sie erst unter einer Vorverformung, also einer Durchmesserreduzierung des Haptikringes von 0,03 mm - 0,5 mm, vorzugsweise 0,05 mm - 0,3 mm, ihren angedachten Auslegungspunkt für den Fernpunkt erreicht.

Die erforderliche Anpassung an die Größe des individuellen Patientenauges bezieht sich bei der Ziliarintraokularlinse nur auf den erforderlichen Durchmesser. Bei einer Kapselintraokularlinse ergibt sich die zusätzliche Schwierigkeit, dass abhängig vom Patienten die axiale Breite des Kapselsacks sehr stark variieren kann, da sich die natürliche Linse mit zunehmendem Alter verdickt.

Vorzugsweise wird die Intraokularlinse aus zwei Halbschalen gefertigt, die mittels Kleben oder Schweißen gefügt und somit stoffschlüssig miteinander verbunden werden.

Allerdings können die Intraokularlinsen auch aus zwei Halbschalen zusammengesetzt, somit zweistückig sein, und form- oder kraftschlüssig verbunden sein. Nach einer weiteren bevorzugten Ausführungsform der Intraokularlinse ist daher vorgesehen, dass die Spitzen der Haptiken von einem dünnen Band oder einer Membran umfasst sind. Ein solches Band schränkt die Kontraktionsfähigkeit der Haptik nicht ein. Die Innenseite des Bandes zwischen den Haptikspitzen ist vorzugsweise ebenso wie die Seitenflächen der Haptiken mit antiproliferativen Substanzen beschichtet, damit in die Durchbrechungen keine Zellen einwachsen können.

Die Haptiken sind vorzugsweise gleichmäßig und gleichförmig über den Umfang der Optik verteilt und die Basen der Haptiken liegen aneinander. Die Basen der Durchbrechungen zwischen den Haptiken müssen dabei nicht bis auf die Optik reichen, womit die Tiefe der Durchbrechungen kleiner sein kann als die radiale Länge der Haptiken. Nach einer weiteren Ausführungsform der vorliegenden Erfindung variieren die Breiten der Durchbrechungen an den Basen der Haptiken und/oder die Breiten der Basen der Haptiken selbst, um die Optik ungleichmäßig zu verformen und damit Abbildungsfehler, wie beispielsweise eine Hornhautverkrümmung des Patienten, auszugleichen. Für diesen Ausgleich, aber auch unabhängig hiervon, ist vorgesehen, dass die einzelnen Haptiken einer Intraokularlinse unterschiedliche Formen oder unterschiedliche von der Optik abweichende Elastizitätsmodule aufweisen. Zudem kann die Ausgestaltung der Haptiken der anterioren gegenüber der posterioren Intraokularlinsenhalbschale hinsichtlich ihrer Formgebung, Breite und Höhe voneinander abweichen, um so eine für die Akkommodation optimale Anpassung bezüglich Elastizität und Verformbarkeit zu erzielen.

Es wurde bereits beschrieben, dass mit dieser Erfindung eine symmetrische Verformung einer oder mehrerer Optiken der Intraokularlinse angestrebt wird. Hierzu sind die Haptikelemente vorzugsweise äquiangular mit der Optik verbunden. Eine symmetrische Verformung der Optik oder Optiken kann jedoch auch hinreichend erzielt werden, wenn eine Mindestzahl von Haptikelementen vorhanden ist und diese nicht genau äquiangular verteilt sind und dabei genau gleiche Basenbreiten haben. Beispielsweise könnten auch bei einer Intraokularlinse mit zwölf Haptikelementen die Elemente 3, 5 und 7 durch eine weitere etwas dünnere strichförmige Ausnehmung halbiert werden und dennoch würde eine hinreichend gleichmäßige und rotationssymmetrische Verformung der Optik erzielt werden können. Bei 12 Haptikelementen spielt die exakte Einhaltung der Äquiangularität der Haptikelemente keine entscheidende Rolle. Wichtig ist, dass die Basen der Haptikelemente über nahezu den gesamten Umfang an die Optiken angebunden sind. Sind dagegen beispielsweise nur 3 Haptikelemente über den Umfang der Optik verteilt, so sollten diese nur wenig von der Äquiangularität und gleichgeformten Haptikelementen abweichen.

Die Kanten der Haptiken sind vorzugsweise eckig bzw. scharf ausgebildet. Allerdings können auch abgerundete Eckkanten vorgesehen sein, insbesondere an den Innenkanten der Haptiksegmente zum Hohlraum der Intraokularlinse. Daher können die Haptiken im Querschnitt neben der bevorzugten Rechteckform beispielsweise auch kreis- oder ellipsenförmig sein. Die Oberfläche der Haptiken ist nach einer bevorzugten Ausführungsform strukturiert ausgebildet oder mit einer biologisch aktiven Beschichtung versehen, womit die Gefahr eines Nachstars oder einer Bakterien-Adhäsion reduziert bzw. vermieden wird. Als Beschichtungswirkstoffe sind vorzugsweise Polysaccharid-Beschichtungen, Heparin, Hyaluronan oder andere Wirkstoffe vorgesehen.

Zur Vermeidung von Streulicht und Blendung, was insbesondere in der Nacht vorkommt, sind die Haptiken vorzugsweise diffus, eingefärbt, undurchsichtig, dotiert oder oberflächenstrukturiert ausgebildet.

Ferner können die Haptiken mit einem Label, einer Identifizierung, einem Produktcode oder einer Seriennummer versehen sein.

Die Formen der anterioren und posterioren Optiken oder ggf. weiteren Optiken können bi-konvex, plan-konvex, planparallel, meniskus, konkav-konvex oder andere Linsenformen aufweisen. Die beiden Optiken können auch unterschiedliche Durchmesser und/oder unterschiedliche Brechungsindexe besitzen. Wenn die anterioren oder posterioren Optiken identische Brechzahlen haben wie die Füllung, können die Innenflächen der anterioren oder posterioren Optiken beliebig geformt sein und von klassischen Optikformen abweichen.

Die Optik oder Optiken der Intraokularlinse dieser Erfindung, insbesondere deren Außenflächen sind vorzugsweise asphärisch geformt, d.h. sie weichen von der Kugelform ab. Die Krümmungsradien der Optiken nehmen von der zentralen optischen Achse zum Rand der Optik vorzugsweise um mehr als 20% und besonders bevorzugt um mehr als 50% zu. In bestimmten Auslegungen kann die Radienzunahme auch 100% oder mehr als 300% betragen.

Nach einer weiteren bevorzugten Ausführungsform der Intraokularlinse ist vorgesehen, dass der Haptikring zwei- bzw. dreistückig mit den Optiken verbunden ist, wobei die Optiken mit den Haptiken form- und/oder kraftschlüssig von dem Haptikring gehalten werden. Hiermit ist die Intraokularlinse kleiner ausgestaltet und leichter in das Auge einführbar. Nach einer besonders bevorzugten Ausführungsform ist dabei vorgesehen, dass der Haptikring aus mehreren Haptikringsegmenten zusammengesetzt ist, die vorzugsweise über eine Membran miteinander verbunden sind. Die Membranen können außen, innen oder auch seitlich des Rings angeordnet sein. Vorzugsweise sind zwischen den Haptikringsegmenten und den Haptiken eine umlaufende Nut und ein entsprechender Rand für einen festeren und präziseren Sitz vorgesehen, wobei andere formschlüssige Verbindungen selbstverständlich auch denkbar sind. Alternativ hierzu ist vorgesehen, dass der Haptikring aus Haptikringsegmenten zusammengesetzt ist, wobei die einzelnen Haptikringsegmente durch einen Faden zusammengehalten werden. Hierdurch entsteht eine Art Kette.

Weiterhin könnte grundsätzlich ein durchgehender Haptikring (ohne einzelne Segmente) ähnlich dem zuvor beschriebenen dünnen Band verwendet werden. Vorzugsweise sollte der Ring hierbei in zirkumferentieller Richtung elastisch bzw. komprimierbar und in radialer Richtung weitgehend nicht komprimierbar sein. Dadurch kann die Kraft des Ziliar möglichst effektiv übertragen werden, zumal die Kontraktion, d.h. die Änderung des Ziliardurchmessers während Nahakkommodation auf wenige zehntel Millimeter begrenzt ist. Alternativ könnte der Haptikring beispielsweise auch nur an einer Stelle geteilt sein.

Die aufgezeigten Konstruktionen der Intraokularlinse können auch als reine Optik-Shift Intraokularlinsen genutzt werden, bei denen also die Optiken nur auf ihrer Achse verschoben und nicht verformt werden. Zudem könnte die Intraokularlinse sowohl in den Kapselsack oder als auch mittelbar an den Ziliar gekoppelt implantiert werden.

Als Werkstoffe für die Intraokularlinse kommen verschiedene Materialien in Frage. Nach einer Ausführungsform der vorliegenden Erfindung ist eine Intraokularlinse vorgesehen, wobei die Intraokularlinse aus einem Silikon, insbesondere einem thermoplastisch zu verarbeitenden Silikon, besteht. Als thermoplastische Silikone sind besonders Polymere aus der Gruppe der Organopolysiloxan/Polyharnstoff/Polyurethan-Blockpolymere verwendbar. Vorzugsweise ist vorgesehen, dass nach dem Fügen das verwendete Silikon vernetzt wird. Allerdings sind auch thermoplastische Polymere und vernetzte thermoplastische Polymere oder auch Elastomere verwendbar, die eine hohe optische Transparenz und vorzugsweise einen hohen Brechungsindex aufweisen. Solche Polymere und Copolymere oder ggf. auch Mischungen daraus können unter anderem die Gruppe der diversen Polyacrylate und Polymethacrylate (wie auch "PHEMA", "PHPMA" etc.), Poly-N-Butyl-Methacrylat) (PBMA), Polyvinyl (Polystyrol, Polyvinylacetat, Poly-N-Vinylpyrrolidone "PNVP"), Ethylenvinylacetate, die Gruppe der Polysiloxane (PDMS), Polyphosphazene, Polyurethane, Polyharnstoffurethane und deren Copolymere einschließlich NH₂- oder OH-terminierten Polyisobutylen Polyurethanen, weitere Hydrogele einschließlich Polyethylenglycol basierter Hydrogele, Polysulfone, auf Styrol-Ethylen-Butylen-Styrol basierte thermoplastische Elastomere (SEBS) oder auch hydrogenierte Styrol-Block-Copolymere, Polystyrol-Block-Isobutylene-Block-Styrol (SIBS), Polypropylen umfassen. Von diesen Polymeren werden vorzugsweise Polystyrol-Block-Isobutylene-Block-Styrol (SIBS) oder Polyurethane auf Basis NH₂- oder OH-terminierten Polyisobutylen verwendet. Das Implantatmaterial muss zudem biokompatibel und biostabil sein. Die Polymere sind daher zur Verbesserung ihrer Bioverträglichkeit oberflächenmodifiziert, was vorzugsweise durch eine Hydrophilisierung durchgeführt wird. Die Polymere können auch wasserdurchlässig sein.

Die Füllung kann insbesondere aus einem superelastischen Polymer oder einer Flüssigkeit bestehen. Neben den zuvor genannten Polymeren kommen insbesondere weitere hydrophile Polymere in Betracht, wie Polyvinylpyrrolidon, Polyvinylalkohol oder Hyaluronsäure. Diese können mit Wasser gemischt werden, insbesondere eignet sich hierzu auch vernetztes Polyvinylpolypyrrolidone (PVPP). Die Flüssigkeit kann Wasser oder eine wässrige Dispersion bzw. kolloid disperse Lösung sein, in denen sich Nanopartikel vorzugsweise aus Polymeren befinden zur Erhöhung des Brechungsindexes. Insbesondere kann die Füllung auch aus halogenierten Kohlenwasserstoffen bestehen, denen Polymethylmethacrylat-Partikel zugesetzt sind. Die Nanopartikel können zudem oberflächenfunktionalisiert oder mit Edelmetallkolloiden (z.B. Gold) beschichtet sein. Weiterhin bieten sich Gold-Sole als Flüssigkeit für die Füllung an.

Zur Erhöhung des Brechungsindex können den Polymeren oder dem Medium der Füllung auch andere Nanopartikel, wie z.B. Titandioxid beigegeben werden. Vorzugsweise kann den Polymeren Nanogold hinzugefügt oder an sie chemisch (kovalent) gebunden werden. Durch das Gold erhält die Intraokularlinse antibakterielle Eigenschaften. Zudem kann das Polymer bzw. die Füllung hierdurch Blaulicht filtern, was als UV-Barriere die Netzhaut schützt.

Der Elastizitätsmodul des Polymers beträgt nach einer vorteilhaften Ausführungsform weniger als 1 N/mm². Der Elastizitätsmodul der Schale bzw. Halbschalen der Ziliarintraokularlinse ist ferner bevorzugt kleiner 0,5 N/mm² und vorzugsweise größer 0,02 N/mm². Anzumerken ist, dass insbesondere im Bereich der Haptikarme, an denen die Halbschalen zusammengeführt sind, der Elastizitätsmodul gegenüber den zuvor aufgeführten Werten höher sein kann. Der Elastizitätsmodul der Füllung ist vorzugsweise kleiner 0,05 N/mm² und besonders bevorzugt kleiner 0,01 N/mm². Bei einer Ausführung der Intraokularlinse als Kapselintraokularlinse kann der Elastizitätsmodul der Schalen niedriger sein als die zuvor angegebenen Werte für die Schale.

Die erfindungsgemäße Intraokularlinse ist auch für weitere technische Anwendungen einsetzbar, wie beispielsweise als stufenlos fokussierbare Optik für 3D-Endoskope, mit integrierter Kamera ausgestattete PC-Monitore für qualitativ hochwertige Videokonferenzen oder Autofokus-Objektive im Low-Cost-Sektor. Die Akkommodation könnte hier über radial wirkende Stellglieder geregelt werden, wie beispielsweise mit einem mit Luft oder Flüssigkeit gefüllten Schlauch.

Eingangs wurde bereits angedeutet, dass die vorliegende Erfindung ferner ein Verfahren zur Implantation einer Intraokularlinse mit einer Optik, einer Haptik sowie einer Füllung betrifft. Erfindungsgemäß ist hierzu vorgesehen, dass die Intraokularlinse zur Verkleinerung des Volumens zusammengefaltet oder -gerollt wird, so dass die Füllung zumindest teilweise in dem Reservoir angeordnet ist und die Füllung nach der Implantation zumindest teilweise aus dem Reservoir in den Hohlraum gedrückt wird. Vorzugsweise ist der expansionsfähige Teil des Reservoirs während der Implantation außerhalb der Intraokularlinse angeordnet. Das erfindungsgemäße Verfahren ist vor allem für Intraokularlinsen geeignet, wie sie im Übrigen in der vorliegenden Anmeldung beschrieben sind.

Schließlich soll eine Intraokularlinse mit einer flüssigen oder gelartigen Füllung geschaffen werden, die in einem kleineren Zustand implantiert werden. Erfindungsgemäß ist daher vorgesehen, dass ein oder mehrere mit der Füllung gefüllte Reservoire über einen Schlauch vorzugsweise lösbar mit dem Hohlraum verbunden sind, so dass bei einer Volumenänderung des Hohlraums während der Akkommodation oder bei dem Zusammenfalten der Intraokularlinse für die Implantation die Füllung in das Reservoir überführbar ist und umgekehrt.

Wie auch die nachfolgenden Figuren zeigen, wird durch das Verfahren das Volumen der Intraokularlinse für den Implantationsvorgang deutlich reduziert. Dies wird insbesondere durch den Aufbau der Intraokularlinse bestehend aus relativ dünner Schale und großer Füllung ermöglicht. Bezieht man bei der Berechnung der Volumenänderung der Linse das Volumen auf den Teil der Intraokularlinse ohne die Haptiken, d.h. auf den Durchmesser am Äquator der Optiken bzw. des Hohlraumes, so lässt sich das Volumen um mehr als 20%, bevorzugt um mehr als 30% und besonders bevorzugt um mehr als 40% reduzieren, wenn man die Linse abflacht und die Füllung in das oder die Reservoire drückt. Vorteilhaft ist bei dieser Anordnung und Verfahren, dass man im Normalbetrieb, also nach Implantation, die Reservoire vorzugsweise mit sehr kleinem Durchmesser posterior dem Haptikring und entlang des Haptikringes ringförmig anordnen kann. Die Reservoire stören dadurch den optischen Bereich der Intraokularlinse nicht. Für den Implantationsvorgang kann sich der Durchmesser des Schlauches durch Aufnahme der Füllung bis zum 8-fachen seines Durchmessers aufblähen.

Wäre beispielsweise das Reservoir im Haptikring integriert, also der Haptikring das Reservoir selbst, dann müsste der Haptikring "für das Aufblähen" relativ elastisch bzw. weich ausgelegt werden, jedoch wäre der Haptikring dann in radialer Richtung auch leicht komprimierbar bzw. zu nachgiebig, so dass der Haptikring die Kräfte des Ziliar nur unzureichend übertragen könnte und so enorme Verluste für die Verformung der Intraokularlinse während der Akkommodation entstehen würden.

Eine weitere Besonderheit in der Auslegung des Reservoirs ergibt sich, wenn das Reservoir als Volumenausgleich während der Akkommodation dient. Üblicherweise ist dann das Volumen der Füllung im Nahzustand etwas größer als im Fernzustand der Intraokularlinse. Die Volumenmenge der Füllung wird nun vorzugsweise gleich oder kleiner dem kleineren Volumen, üblicherweise für den Fernzustand bemessen. Damit für den Nahzustand während der Akkommodation das Volumen der Füllung in der Intraokularlinse zunehmen kann, zieht sich der Schlauch in eine flache Form, z.B. von einem kreisförmigen Querschnitt in eine Ellipsenform, zusammen und reduziert damit sein Volumen. Es entsteht also eine Art Saugwirkung aus der Linse auf das Reservoir. Dadurch dass sich das Reservoir bzw. der Schlauch aus seinem Normal-/Fertigungszustand zusammenfaltet und kontrahiert, wird vermieden, dass der andernfalls nötige hohe Anfangswiderstand zum Ausdehnen des Schlauches überwunden werden muss. Dieser Anfangswiderstand kann nicht durch den Akkommodationsvorgang überwunden werden, selbst wenn der Schlauch nur sehr dünne Wandstärken hat. Der Anfangswiderstand zum Aufblähen des Reservoirs kann aber überwunden, wenn die Intraokularlinse für den Implantationsvorgang flach gedrückt und anschließend aufgerollt wird. Angemerkt sei ferner, dass die bevorzugte Auslegung der Menge der Füllung für den kleineren Volumenzustand bzw. Fernzustand auch für eine Intraokularlinse gilt, deren Fertigungszustand im Nahzustand liegt und folglich das Reservoir beim Befüllen der Füllung etwas zusammengedrückt werden muss, um Luft im Reservoir zu vermeiden.

Konkrete Ausführungsformen der vorliegenden Erfindung werden im Folgenden sowie anhand der Figuren erläutert. Es zeigen:
- Fig. 1a, b:: schematische Darstellungen einer implantierten Ziliarintraokularlinse,
- Fig. 2a-3i:: Intraokularlinsen in unterschiedlichen Perspektiven,
- Fig. 4:: eine Intraokularlinse mit verschiedenen Ausnehmungen,
- Fig. 5a,b:: Explosionsdarstellungen verschiedener Intraokularlinsen und
- Fig. 6a-l:: diverse Intraokularlinsen mit Reservoiren.

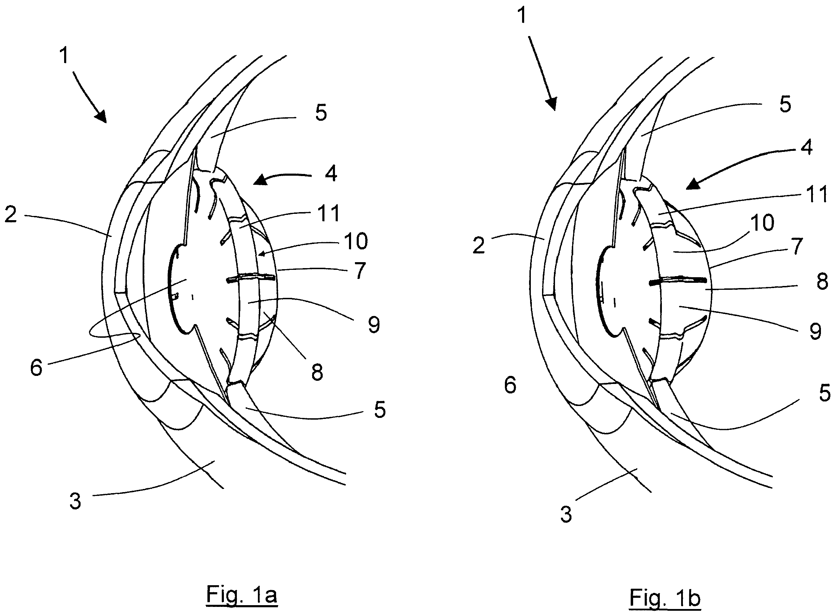

Die Fig. 1a und b zeigen je eine Schnittansicht eines Auges 1 mit der Hornhaut 2 und der Lederhaut 3 sowie einer Ziliarintraokularlinse 4. Die Intraokularlinse 4 steht in direktem Kontakt zu dem Ziliarmuskel 5, so dass die Kontraktionskraft unmittelbar auf die Ziliarintraokularlinse 4 übertragen wird. Um hierbei eine gleichmäßige Verformung der Optik 6,7 erzielen zu können, ist an den Haptiken 8 eine möglichst großflächige Kontaktfläche 9 für den Ziliarmuskel 5 ausgebildet, die sich entlang eines Haptikrings 10 erstreckt. Um den Haptikring 10 ausreichend kontrahieren zu können, ist der Haptikring 10 im dargestellten Ausführungsbeispiel in Haptikringsegmente 11 unterteilt, die im unbelasteten Zustand geringfügig voneinander beabstandet sind. Ferner sind die Haptiken am Übergang zu der Optik 6, 7 miteinander verbunden. Weitere Details auch zu verschiedenen Ausführungen der Intraokularlinsen 4 werden im Folgenden anhand der Figuren beschrieben. Es sei darauf hingewiesen, dass zwischen Ziliar und Kontaktfläche der Intraokularlinse auch der Kapselsack bzw. Teile davon und/oder der Zonularfasern liegen können.

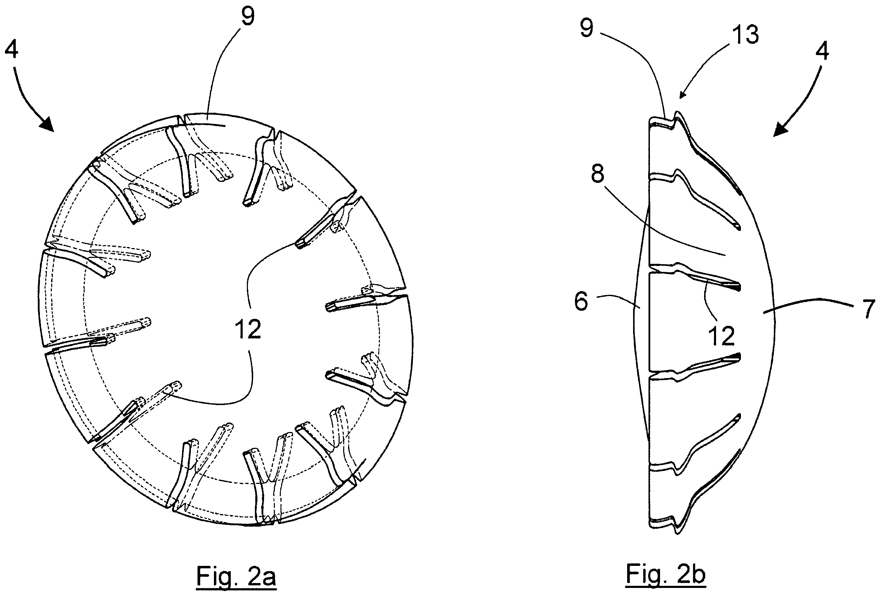

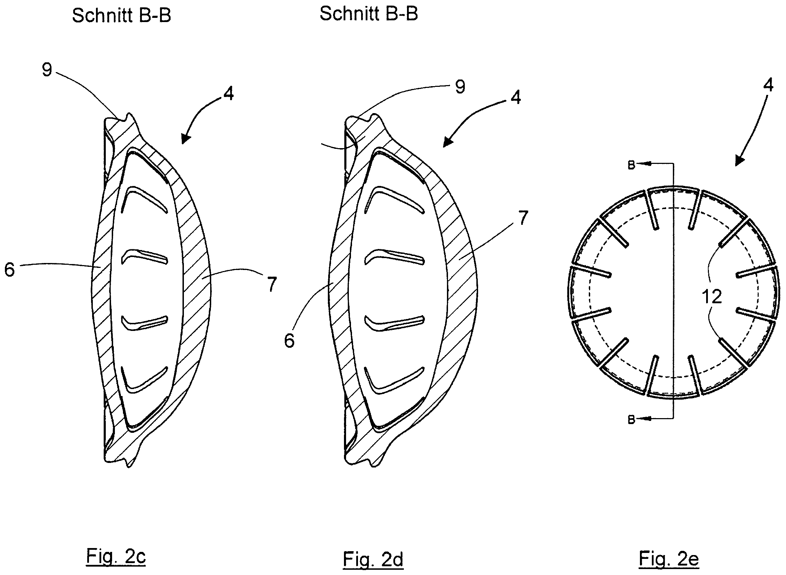

Die Figuren 2a bis e zeigen ein konkretes Ausführungsbeispiel einer Intraokularlinse 4 in unterschiedlichen Perspektiven. In der perspektivischen Ansicht (Fig. 2a) ist deutlich zu erkennen, dass die anteriore und posteriore Optik 6, 7 über - im dargestellten Ausführungsbeispiel - 12 Haptikelemente 8 miteinander verbunden sind. Die Haptikelemente 8 sind durch radial geführte strichförmige Ausnehmungen 12 voneinander beabstandet, so dass sowohl an der Kontaktfläche 9 als auch am Übergang zwischen den Haptikelementen 8 und der Optik 6, 7 eine größtmögliche Fläche zur Kraftübertragung entsteht. Damit ein Verrutschen der Intraokularlinse 4 innerhalb des Ziliar 5 vermieden wird, ist die Kontaktfläche im Querschnitt V-förmig ausgebildet (Pfeil 13).

Die bei der Akkommodation unterschiedlich geformten Intraokularlinsen 4 sind schematisch in den Figuren 2c und d dargestellt. Im flachen und unverformten Zustand (Figur 2c) dient die Intaokularlinse 4 der Nahsicht. Bei der Fernsicht hingegen kontrahiert der Ziliar 5 und die Optiken 6,7 werden zu größeren Krümmungen hin verformt (Figur 2d).

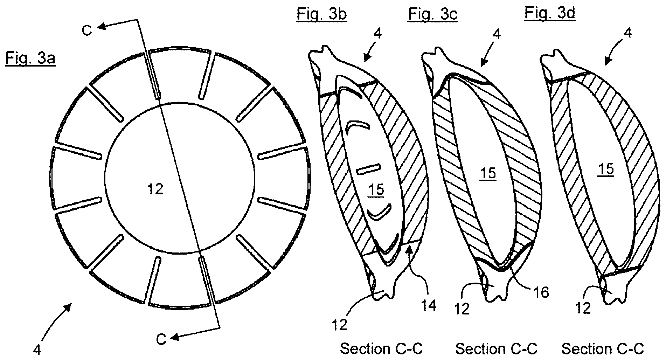

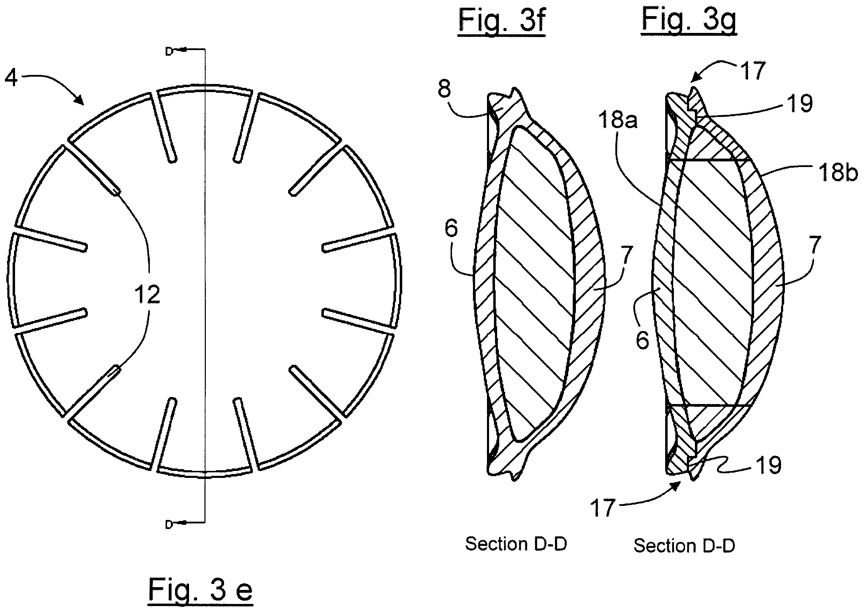

Bei denen in den Figuren 1a bis 3g dargestellten Ausführungsbeispielen sind die Ausnehmungen 12 strichförmig ausgebildet. Derartige Ausnehmungen 12 sind vergleichsweise einfach in die Intraokularlinsen einzubringen, wobei im Wesentlichen drei verschiedene Möglichkeiten vorgesehen sind, bis wohin die Ausnehmungen 12 eingebracht bzw. bzw. eingeschnitten sind. Figur 3b zeigt eine Variante, bei der die Ausnehmung 12 bis an den Rand des optischen Bereichs der Intraokularlinse 4 geführt ist, wodurch der Hohlraum 15 zwischen den Optiken 6,7 geöffnet wird. Demgegenüber ragt zwar die Ausnehmung 12 bei der Ausführungsform nach Figur 3c ebenfalls bis an den Rand der Optik 6, 7, wobei jedoch eine dünne Membran 16 nicht durchbrochen wird, und der Hohlraum 15 damit nicht geöffnet wird. Schließlich zeigt Figur 3d eine Ausgestaltung, wonach die Ausnehmungen 12 innerhalb der Haptik 8 enden, womit der Hohlraum 15 ebenfalls verschlossen bleibt.

Die Intraokularlinsen 4 können einstückig ausgebildet sein oder aus zwei Halbschalen 18a, 18b bestehen, die mit den Haptiken 8 form-, kraft- oder stoffschlüssig verbunden sind (Figur 3f bis 3g).

Figur 3g zeigt insbesondere eine mögliche Teilung der beiden Intraokularlinsenhalbschalen 18a, 18b, die ein konzentrisches Zusammenfügen der beiden Halbschalen 18a, 18b vereinfacht und einem in Formschluss ermöglicht, indem die Verbindungsflächen korrespondierende Stufen 19 aufweisen. Durch unterschiedliche Stufenhöhen bei den einzelnen Haptikelementen ist auch ein angulares Ausrichten beider Halbschalen 18a, 18b möglich. Ein angulares Ausrichten der Halbschalen 18a, 18b ist insbesondere bei Intraokularlinsen 4 vorteilhaft, bei denen die Halbschalen 18a, 18b unterschiedliche Elastizitätsmodule besitzen. Die Schnittdarstellung der Fig. 3g zeigt darüber hinaus, dass die Optiken, Haptiken und auch die Füllung aus unterschiedlichen Materialien mit unterschiedlichen Elastizitätsmodulen zusammengesetzt sein können.

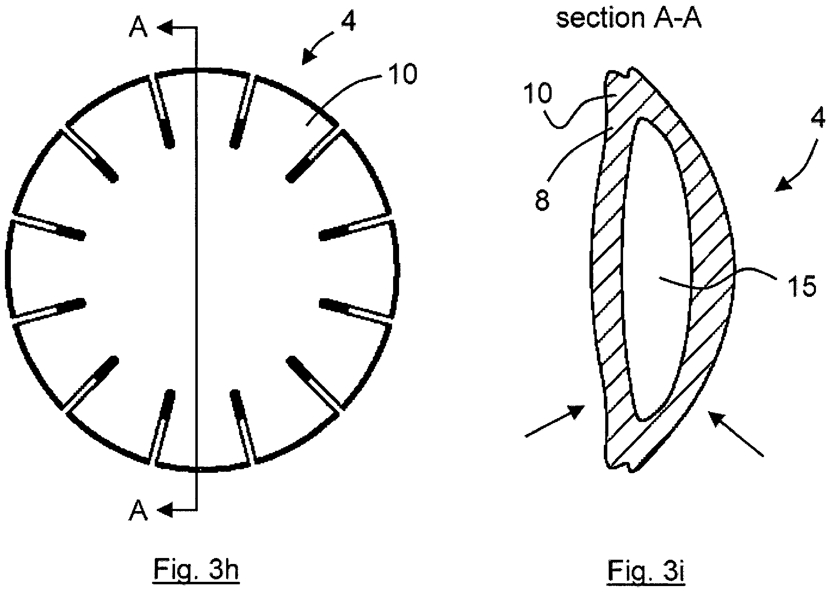

Fig. 3h und 3i zeigen eine Ausführungsform einer Intraokularlinse 4 mit einen Haptikring 10, der im Querschnitt breiter ausgelegt ist und damit gegenüber den vorherigen Ausgestaltungen steifer wird, so dass sich der Haptikring 10 in radialer Richtung weniger verformen kann und damit die Durchmesseränderung des Ziliar effektiver auf die Optiken überträgt. Hierbei liegen die geringsten Wandstärken der Halbschalen nicht mehr im Haptikring 10 bzw. am Äquator des Hohlraumes 15 wie bei den vorherigen Versionen, sondern verlagern sich zur Basis der Haptik 8 bzw. zum Optikrand (siehe Pfeile in Fig. 3i).

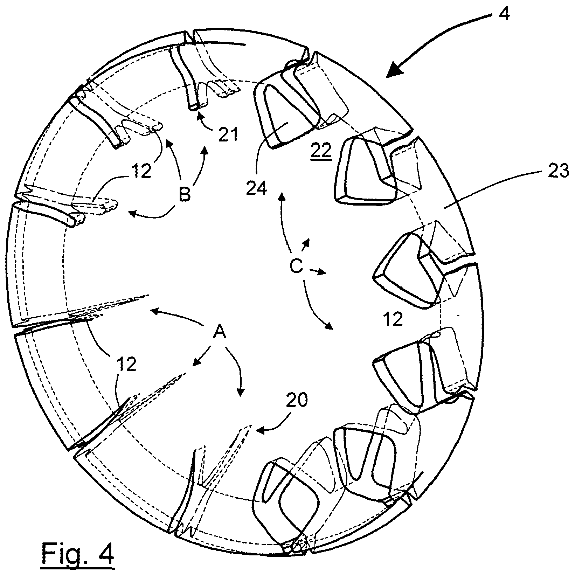

Wie der schematischen Darstellung der Figur 4 zu entnehmen ist, sind unterschiedliche Formen von Ausnehmungen 12 vorgesehen. Die Varianten A und B zeigen strichförmige Ausnehmungen 12, wobei die Seitenflächen der Ausnehmungen 12 bei Variante A spitzwinkelig zusammenlaufen (Pfeil 20), wohingegen bei Variante B ein abgerundeter Übergang vorgesehen ist (Pfeil 21). Eine besonders bevorzugte Geometrie zeigt Variante C. Hiernach bestehen die Haptikelemente 8 aus im Wesentlichen trapezförmigen Teilbereichen, wobei diese auf der der Optik 6, 7 abgewandten Seite spitzwinkelig zusammenlaufen (Bezugszeichen22). Dort bilden die Haptikelemente 8 teilringförmige Haptikringsegmente 23 aus, wobei benachbarte Haptikringsegmente 23 im dargestellten unbelasteten Zustand geringfügig voneinander beabstandet sind. Hierdurch entsteht zwischen zwei Haptikelementen 8 eine tortenstückförmige Ausnehmung (12). Die Haptikelemente mit den Ausnehmungen der Variante C ähneln damit T-förmigen Haptikelementen. Die Haptikelemente mit den strichförmigen Ausnehmungen bilden im übertragenen Sinne trapezförmige Teilbereiche mit stumpfwinkelig auseinanderlaufenden Seiten, an denen die teilringförmigen Haptikringsegmente übergangslos anschließen und bilden insgesamt damit trapezförmige oder strichförmige Haptikelemente.

In den dargestellten Ausführungsbeispielen wird der Hohlraum 15 von den Ausnehmungen 12 geöffnet, so dass Durchbrechungen 24 gebildet werden. Diese Durchbrechungen 24 können mittels einer dünnen Membran verschlossen werden, ohne dass die Elastizität der Intaokularlinse 4 hiervon beeinflusst wird.

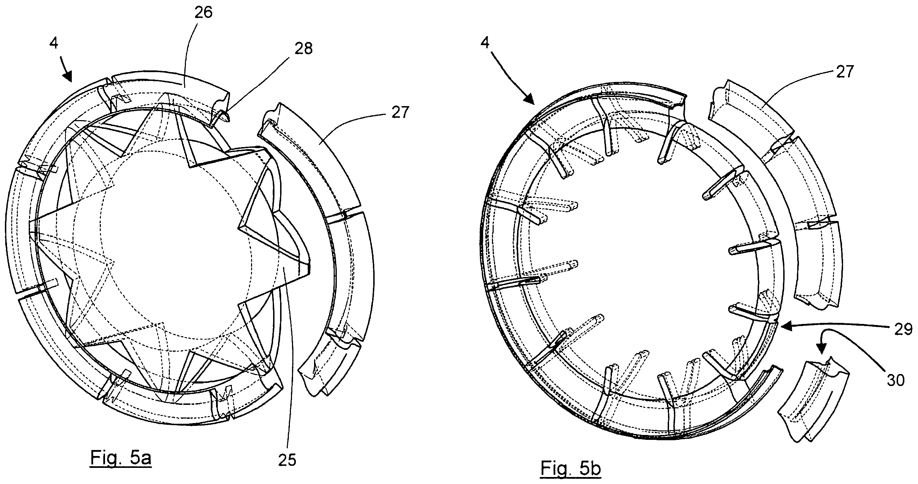

Nach einer weiteren konkreten Ausführungsform der vorliegenden Erfindung ist vorgesehen, dass die Intraokularlinsen 4 geteilt ausgestaltet sind, um sie leichter in das Auge einführen zu können. Die Figuren 5a, b zeigen Ausgestaltungen, bei denen die Ziliarintraokularlinse 4 aus einer vergleichsweise kleinen Intraokularlinse 25 und einem separaten Haptikring 26 besteht. Der separate Haptikring 26 ist durch Durchbrechungen 24 in mehrere Segmente 27 geteilt, die mit Membranen 28 miteinander verbunden sind. Figur 5a zeigt ein Ausführungsbeispiel, bei dem die Membran 28 innen angeordnet ist, wobei bei der Variante gemäß Figur 5b die Membran 28 außen oder seitlich vorgesehen ist, wie es bei dem herausgezogenen dreier-Segment der Explosionsdarstellung gezeigt ist. Ferner können an der Kontaktfläche zwischen dem separaten Haptikring 26 und der Intraokularlinse 25 nut- oder federförmige Konturen 29, 30 vorgesehen sein, um einen stabilen Halt zu schaffen. Die innen liegende Intraokularlinse 25 kann ebenfalls unterschiedlich ausgestaltet sein, wobei die oben beschriebenen Ausführungsformen bevorzugt sind.

Nach einer konkreten Ausführungsform der vorliegenden Erfindung weist der Hohlraum 15, der zwischen den Halbschalen 18a, 18b gebildet wird, eine Füllung 32 auf, die ggf. innerhalb eines Säckchens bzw. einer den Hohlraum abgrenzenden Membran 31 ruht. Bei der Akkommodation werden nicht nur die Optiken 6, 7 verformt, sondern auch die Oberfläche des Säckchen bzw. der Membran 31 und der Füllung 32, was sich auf die Brechkraft unterschiedlich auswirkt. Um eine solche Intraokularlinse 4 mit einer Füllung 32 in das Auge implantieren zu können, und um eine Volumenänderung des Hohlraums 15 bei der Akkommodation zu ermöglichen, sind nach einer konkreten Ausführungsform der Erfindung Reservoire 33 vorgesehen, die lösbar oder unlösbar mit dem Hohlraum 15 hydraulisch verbunden sind.

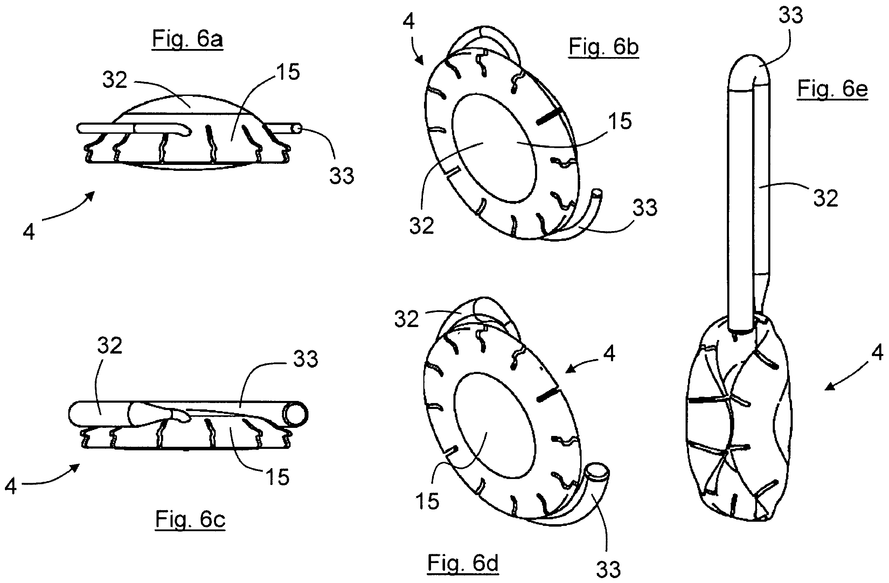

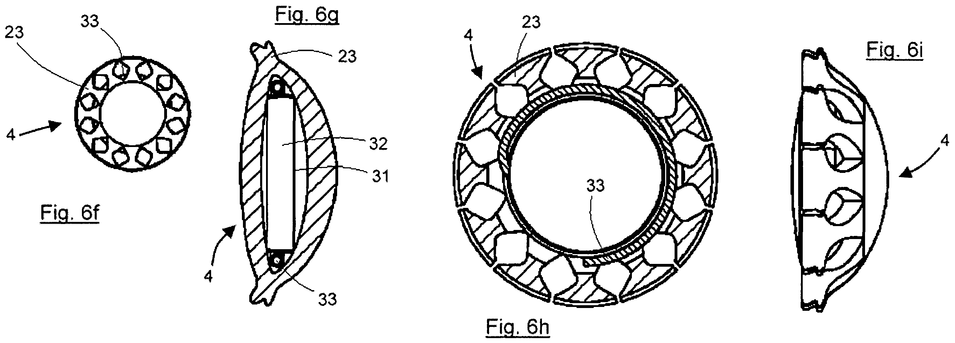

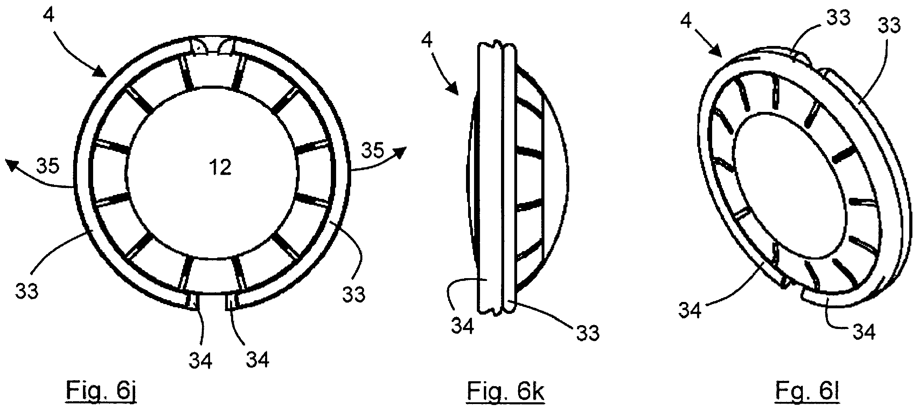

In den Figuren 6a bis 6l sind verschiedene Ausgestaltungen solcher Intraokularlinsen 4 mit einer Füllung 32 und mindestens einem Reservoir 33 dargestellt. In der Ausführungsform nach Figur 6a bis 6e ist ein einzelnes Reservoir 33 vorgesehen, das schlauchförmig außerhalb der Intraokularlinse 4 angeordnet ist. Die Figuren 6a, b zeigen die Situation, bei der die Füllung 32 nahezu vollständig in den Hohlraum 15 angeordnet ist. Demgegenüber ist in Figur 6c, d dargestellt, wie sich die Füllung 32 innerhalb des Reservoirs 33 befindet und auch die Intraokularlinse 4 vergleichsweise flach ist. Aufgrund einfacherer Herstellung besitzt das schlauchförmige Reservoir 33 wie in Fig. 6a, b dargestellt vorzugsweise einen gleichmäßigen Durchmesser. Hierbei weist der Teil des Schlauches nahe der Intraokularlinse jedoch eine größere Wandstärke als der Rest des Schlauches auf, so dass sich nur der Teil des Schlauches mit der geringeren Wandstärke bei Aufnahme der Füllung aufbläht (Fig. 6c, d). In einem kurzen Übergangsbereich gleichen sich die unterschiedlichen Wandstärken an. Selbstverständlich kann das schlauchförmige Reservoir auch über seine Länge unterschiedliche Durchmesser aufweisen.

Vor der Implantation kann die Füllung 32 in der erforderlichen Menge von dem Hohlraum 15 in das Reservoir 33 gedrückt werden, so dass die Intraokularlinsen 4 zusammengerollt bzw. zusammengefaltet werden können (Figur 6e). Das Reservoir 33 verläuft weiterhin außerhalb der Intraokularlinse 4, so dass die Implantation bei einem minimalen Schnitt erfolgen kann.

Alternativ zu der Ausführungsform, bei der das Reservoir 33 außerhalb der Intraokularlinse 4 verläuft, kann das Reservoir 33 auch innerhalb der Intraokularlinse 4 angeordnet sein (Figur 6f bis 6i). Hierzu ist es schlauchförmig ausgebildet und zwischen den Haptikelementen 8 verlegt, so dass die optischen Eigenschaften hierdurch unbeeinflusst bleiben.

Schließlich sind auch Ausführungsformen vorgesehen, bei denen zwei oder mehr Reservoire 33 die Füllung 32 aufnehmen. Die Figuren 6j bis 6l zeigen eine Ausgestaltung mit zwei Reservoiren 33, die die Intraokularlinse 4 um jeweils 180° umgreifen. Zudem weisen die Intraokularlinsen 4 einen weiteren Ring bzw. zwei Ringelemente 34 auf, die ebenfalls die Intraokularlinsen 4 jeweils um 180° umgreifen. Die Ringelemente 34 sind mit den Reservoiren 33 und einem Haptikelement 8 verbunden und können zusammen mit den Reservoiren 33 in Pfeilrichtung 35 verschwenkt werden, um die Intraokularlinse 4 für die Implantation kompakter auszugestalten. Die Ringelemente 34 können über die Haptikelemente 8 gleiten und damit die durch den Ziliar bedingte Durchmesseränderung ausgleichen.

## Patentansprüche

1. Ziliarintraokularlinse mit mindestens einer Optik (6, 7) und einer Haptik,
**dadurch gekennzeichnet, dass**
die Haptik aus mehreren Haptikelementen (8) besteht, die vorzugsweise äquiangular mit der Optik (6, 7) verbunden sind, wobei
a) die Haptikelemente (8) einen in einer Draufsicht im Wesentlichen trapezförmigen Teilbereich besitzen und die Basen zweier benachbarter Haptikelemente (8) am Übergang zu der Optik (6, 7) miteinander verbunden sind und
b) die Haptikelemente (8) an der der Optik (6, 7) abgewandten Seite der trapezförmigen Teilbereiche je ein teilringförmiges Haptikringsegment (11, 23) besitzen, die eine zylinderförmige Außenkontur bilden und im Querschnitt V-förmig ausgestaltet sind, so dass sich die Ziliarintraokularlinse der Kontur des Ziliar formschlüssig anpasst, um ein Verrutschen der Optik oder der Intraokularlinse innerhalb des Ziliar zu vermeiden, wobei die Haptikringsegmente (11, 23) zweier benachbarter Haptikelemente (8) im unbelasteten Zustand geringfügig voneinander beabstandet sind,
so dass zwischen zwei benachbarten Haptikelementen eine im Wesentlichen tortenstück- oder strichförmige Ausnehmung ausgebildet ist, wobei im unbelasteten Zustand die kumulierte Breite der Ausnehmungen am Außendurchmesser des Haptikrings weniger als 25 % des Umfangs beträgt.

2. Intraokularlinse nach Anspruch 1, **dadurch gekennzeichnet, dass** die Intraokularlinse (4) mindestens eine anteriore und eine posteriore Optik (6, 7) aufweist, die über die Haptik miteinander verbunden sind, wobei durch die anteriore und posteriore Optik (6, 7) sowie die Haptik ein Hohlraum (15) gebildet wird und zumindest der optische Bereich des Hohlraums (15) eine Füllung aufweist, wobei die Füllung vorzugsweise flüssig, gelförmig oder gasförmig ist und vorzugsweise Nanopartikel aufweist und wobei die Füllung vorzugsweise eine höhere Brechzahl als das Kammerwasser aufweist.

3. Intraokularlinse nach einem der Ansprüche 1 oder 2, **dadurch gekennzeichnet, dass** der Bereich des Hohlraums (15) im Bereich der Haptik und/oder der Ausnehmungen teilweise oder vollständig mit der Füllung gefüllt sind, wobei die Füllung flüssig, gelförmig oder gasförmig ist und vorzugsweise Nanopartikel aufweist und wobei die Füllung eine höhere Brechzahl als das Kammerwasser aufweist.

4. Intraokularlinse nach einem der Ansprüche 1 bis 3, **gekennzeichnet durch** ein oder mehrere Reservoire (33), die mit der Füllung befüllt sind und die über einen Schlauch vorzugsweise lösbar mit dem Hohlraum (15) verbunden sind, so dass bei einer Volumenänderung des Hohlraums (15) während der Akkommodation oder bei dem Zusammenfalten der Intraokularlinse (4) für die Implantation die Füllung in das Reservoir (33) überführbar ist und umgekehrt, wobei das Reservoir (33) vorzugsweise schlauchförmig ausgebildet ist und vorzugsweise
a) über ein Mikroventil oder eine Kanüle bzw. Hohlnadel mit dem Hohlraum (15) verbunden ist oder
b) über einen Schlauch mit dem Hohlraum (15) verbunden ist, der schmelzend abtrennbar ist.

5. Intraokularlinse nach Anspruch 4, **dadurch gekennzeichnet, dass** die Verbindung zwischen dem Reservoir (33) und dem Hohlraum (15) an der Membran, der Haptik oder außerhalb des optischen Bereichs der Optik angeordnet ist.

6. Intraokularlinse nach einem der Ansprüche 1 bis 5, **dadurch gekennzeichnet, dass** die Füllung innerhalb des Hohlraums (15) zumindest teilweise von einer Membran umschlossen ist, wobei die Membran vorzugsweise wasserdurchlässig ist.

7. Intraokularlinse nach einem der Ansprüche 1 bis 6, **dadurch gekennzeichnet, dass** die Haptik und/oder Haptikringsegmente (11, 23) je ein anteriores und ein posteriores Teilstück aufweisen, die am Äquator miteinander verbunden sind.

8. Intraokularlinse nach einem der Ansprüche 1 bis 7, **dadurch gekennzeichnet, dass** die anteriore Optik (6) oder die posteriore Optik (7) im Randbereich genauso dünn oder dünner ausgebildet sind als auf der zentralen optischen Achse.

9. Intraokularlinse nach einem der Ansprüche 1 bis 8, **dadurch gekennzeichnet, dass** die Durchbrechungen durch eine Membran oder eine Haut verschlossen sind.

10. Intraokularlinse nach einem der Ansprüche 1 bis 9, **dadurch gekennzeichnet, dass** die Ausnehmungen vom Äquator der Haptik radial geführt sind und
a) innerhalb der Haptik enden,
b) bis zum Äquator der Optiken (6, 7) führen oder
c) über den Äquator der Optiken (6, 7) hinaus führen.

11. Intraokularlinse nach einem der Ansprüche 1 bis 10, **dadurch gekennzeichnet, dass** im unbelasteten Zustand die kumulierte Breite der Ausnehmungen am Außendurchmesser des Haptikrings 2-15% des Umfangs beträgt und/oder das die kumulierten Abstände der Basen der Haptikelemente weniger als 25%,vorzugsweise weniger als 15%, des Umfangs des Außendurchmessers des Haptikrings beträgt.

12. Intraokularlinse nach einem der Ansprüche 1 bis 11, **dadurch gekennzeichnet, dass** zur maximalen Kraftübertragung der Haptikring im maximal belasteten Zustand vollumfänglich mit dem Ziliar in Anlage ist.

13. Intraokularlinse nach einem der Ansprüche 1 bis 12, **dadurch gekennzeichnet, dass** die Basen der Haptiksegmente bündig mit mindestens einer der Optiken verbunden sind.

## Claims

1. Ciliary intraocular lens with at least one optic (6, 7) and one haptic,
**characterized in that**
the haptic is composed of a plurality of haptic elements (8), which are preferably connected with the optic (6, 7) in equiangular manner, wherein
a) the haptic elements (8) have an essentially trapezoidal portion in a plan view and the bases of two adjacent haptic elements (8) are connected to each other at the transition to the optic (6, 7) and
b) the haptic elements (8) each have a partially annular haptic ring segment (11, 23) on the side of the trapezoidal portions facing away from the optic (6, 7), which haptic ring segments (11, 23) form a cylindrical outer contour and are V-shaped in cross-section, so that the ciliary intraocular lens adapts form-fitting to the contour of the ciliary in order to prevent the optics or the intraocular lens from slipping within the ciliary, wherein the haptic ring segments (11, 23) of two adjacent haptic elements (8) being slightly spaced apart from one another in the unloaded state,
so that an essentially cake-piece or line-shaped recess is formed between two adjacent haptic elements, whereby in the unloaded state the cumulative width of the recesses on the outer diameter of the haptic ring is less than 25% of the circumference.

2. Intraocular lens according to claim 1, characterized that the intraocular lens at least has an anterior and a posterior optic (6, 7), which are connected to each other via the haptic, wherein a cavity (15) is formed by the anterior and posterior optic (6, 7) as well as the haptic and at least the optic area of the a cavity (15) has a filling, whereby the filling preferably is liquid, gel-like, or gaseous and preferably comprises nano-particles and whereby the filling preferably has a higher refractive index than the aqueous humour.

3. Intraocular lens according to one of claim 1 or 2 **characterized in that** the area of the cavity (15), which is bounded by the haptic and/or the recesses is occupied partially or completely by the filling, whereby the filling is liquid, gel-like, or gaseous and preferably comprises nano-particles and whereby the filling preferably has a higher refractive index than the aqueous humour.

4. Intraocular lens according to one of claims 1 to 3, **characterized by** one or more reservoirs (33) which are filled with the filling and which are preferably detachably connected to the cavity (15) via a tube, so that, for a change in volume of the cavity (15) during accommodation or folding of the intraocular lens (4) for implantation, the filling can be transferred into the reservoir (33) and vice versa, wherein the reservoir (33) is preferably tubular and is preferably
a) connected with the cavity (15) via a micro-valve or a cannula or a hollow needle or
b) connected with the cavity (15) via a tube, which is separable by melting.

5. Intraocular lens according to claim 4, **characterized in that** the connection between the reservoir (33) and the cavity (15) is arranged at the membrane, the haptic or outside the optical region of the optic.

6. Intraocular lens according to one of claims 1 to 5, **characterized in that** the filling is at least partially enclosed within the cavity (15) by a membrane, wherein the membrane is preferably permeable to water.

7. Intraocular lens according to one of claims 1 to 6, **characterized in that** the haptic and/or haptic ring segments (11, 23) each have an anterior and a posterior section which are connected to one another at the equator.

8. Intraocular lens according to one of claims 1 to 7, **characterized in that** the anterior optic (6) or the posterior optic (7) at the edge area are formed thin or thinner than at the central optical axis.

9. Intraocular lens according to one of claims 1 to 8 **characterized in that** the openings are closed with a membrane or a skin.

10. Intraocular lens according to one of claims 1 to 9, **characterized in that** the recesses are aligned radially from the equator of the haptic and
a) terminate within the haptic,
b) lead up to the equator of the optic (6, 7) or
c) extend beyond the equator of the optic (6, 7).

11. Intraocular lens according to one of claims 1 to 10, **characterized in that** in the unloaded state the cumulative width of the recesses on the outer diameter of the haptic ring is 2-15% of the circumference and/or that the cumulative spaces of the bases of the haptic elements is less than 25%, preferably less than 15%, of the circumference of the outside diameter of the haptic ring.

12. Intraocular lens according to one of claims 1 to 11, **characterized in that**, for maximum force transmission, the haptic ring in the maximum loaded state is in full circumferential contact with the ciliary.

13. The intraocular lens according to one of claims 1 to 12 **characterized in that** the bases of the haptic segments are connected flush with at least one of the optics.

## Revendications

1. Lentille intraoculaire ciliaire avec au moins une optique (6, 7) et une haptique,
**caractérisée par le fait que**
l'haptique est constituée d'une pluralité d'éléments haptiques (8) qui sont de préférence reliés de manière équi-angulaire au système optique (6, 7), où
a) les éléments haptiques (8) ont une région partielle qui est essentiellement trapézoïdale dans une vue en plan, et les bases de deux éléments haptiques adjacents (8) sont reliées l'une à l'autre à la transition vers l'optique (6, 7), et
b) les éléments haptiques (8) présentent chacun un segment annulaire haptique partiellement annulaire (11, 23) sur le côté des sous-régions trapézoïdales faisant face à l'optique (6, 7), qui forme un contour extérieur cylindrique et sont agencés en forme de V en coupe transversale, de sorte que la lentille intraoculaire cristalline s'adapte positivement au contour du ciliaire afin d'éviter que l'optique ou la lentille intraoculaire ne glisse dans le ciliaire, les segments annulaires haptiques (11, 23) de deux éléments haptiques adjacents (8) étant légèrement espacés l'un de l'autre à l'état non chargé,
de telle sorte qu'entre deux éléments haptiques adjacents se forme un évidement sensiblement en forme de morceau de gâteau ou de ligne, dans lequel, à l'état non chargé, la largeur cumulative des évidements au diamètre extérieur de l'anneau haptique est inférieure à 25 % de la périphérie.

2. Lentille intraoculaire selon l'exigence 1, **caractérisée en ce que** la lentille intraoculaire (4) présente au moins une optique antérieure et une optique postérieure (6, 7) qui sont reliées entre elles par l'haptique, où l'optique antérieure et l'optique postérieure (6, 7) et l'haptique forment une cavité (15) et au moins la région optique de la cavité (15) présente un remplissage, dans lequel le remplissage est de préférence liquide, sous forme de gel ou gazeux et a de préférence des nanoparticules et dans lequel le remplissage a de préférence un indice de réfraction plus élevé que l'humeur aqueuse.

3. Lentille intraoculaire selon l'une des exigences 1 ou 2, **caractérisée en ce que** l'espace de la cavité (15) dans la région de l'haptique et/ou les évidements sont partiellement ou complètement remplis avec le remplissage, le remplissage étant liquide, sous forme de gel ou gazeux et ayant de préférence des nanoparticules et le remplissage ayant un indice de réfraction plus élevé que l'humeur aqueuse.

4. Lentille intraoculaire selon l'une des exigences 1 à 3, **caractérisée par** un ou plusieurs réservoirs (33) qui sont remplis du remplissage et qui sont de préférence reliés par un tube à la cavité (15), de sorte que, en cas de modification du volume de la cavité (15) pendant le placement ou pendant le pliage de la lentille intraoculaire (4) pour implantation, le remplissage puisse être transféré dans le réservoir (33) et vice versa, le réservoir (33) étant de préférence tubulaire et
a) est de préférence relié à la cavité (15) par une microvalve ou une canule ou une aiguille creuse, ou
b) est de préférence relié à la cavité (15) par l'intermédiaire d'un tuyau flexible, qui peut être séparé par fusion.

5. Lentille intraoculaire selon la exigences 4, **caractérisée en ce que** la liaison entre le réservoir (33) et la cavité (15) est disposée au niveau de la membrane, de l'haptique ou en dehors de la zone optique de l'optique.

6. Lentille intraoculaire selon l'une des exigences 1 à 5, **caractérisée en ce que** le remplissage à l'intérieur de la cavité (15) est entouré au moins partiellement par une membrane, la membrane étant de préférence perméable à l'eau.

7. Lentille intraoculaire selon l'une des exigences 1 à 6, **caractérisée en ce que** l'haptique et/ou les segments annulaires haptiques (11, 23) présentent chacun une section antérieure et une section postérieure qui sont reliées entre elles à l'équateur.

8. Lentille intraoculaire selon l'une des exigences 1 à 7, **caractérisée en ce que** l'optique antérieure (6) ou l'optique postérieure (7) sont conçues pour être aussi fines ou plus fines dans la zone marginale que sur l'axe optique central.

9. Lentille intraoculaire selon l'une des exigences 1 à 8, **caractérisée en ce que** les ouvertures sont fermées par une membrane ou une peau.

10. Lentille intraoculaire selon l'une des exigences 1 à 9, **caractérisée en ce que** les évidements sont guidés radialement depuis l'équateur de l'haptique, et
a) terminent dans l'haptique,
b) mènent jusqu'à l'équateur de l'optique (6, 7) ou
c) sortent par-dessus l'équateur de l'optique (6, 7).

11. Lentille intraoculaire selon l'une des exigences 1 à 10, **caractérisée en ce que**, à l'état non chargé, la largeur cumulée des évidements au diamètre extérieur de l'anneau haptique est de 2 à 15 % de la circonférence et/ou **en ce que** les distances cumulées des bases des éléments haptiques sont inférieures à 25 %, de préférence inférieures à 15 %, du diamètre extérieur de l'anneau haptique.

12. Lentille intraoculaire selon l'une des exigences 1 à 11, **caractérisée en ce que**, pour une transmission de force maximale, l'anneau haptique à l'état de charge maximale est complètement joint au ciliaire.

13. Lentille intraoculaire selon l'une des exigences 1 à 12, **caractérisée en ce que** les bases des segments haptiques sont reliées à fleur avec au moins un des optiques.
